(19) Europäisches Patentamt
European Patent Office
Office européen des brevets

(11) **EP 4 019 943 B1**

(12) **FASCICULE DE BREVET EUROPEEN**

(45) Date de publication et mention
de la délivrance du brevet:
**07.06.2023   Bulletin 2023/23**

(21) Numéro de dépôt: **21217206.8**

(22) Date de dépôt: **22.12.2021**

(51) Classification Internationale des Brevets (IPC):
***G01N 21/17*** *(2006.01)*

(52) Classification Coopérative des Brevets (CPC):
**G01N 21/1702;** G01N 2021/1706

(54) **DISPOSITIF DE DÉTECTION PHOTOACOUSTIQUE COMPORTANT UNE MEMBRANE DE PROTECTION**

FOTOAKUSTISCHE DETEKTIONSVORRICHTUNG MIT EINER SCHUTZMEMBRAN

PHOTOACOUSTIC DETECTION DEVICE COMPRISING A PROTECTIVE MEMBRANE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **24.12.2020   FR 2014113**

(43) Date de publication de la demande:
**29.06.2022   Bulletin 2022/26**

(73) Titulaire: **Commissariat à l'Energie Atomique et
aux Energies
Alternatives
75015 Paris (FR)**

(72) Inventeurs:
• **COUTARD, Jean-Guillaume
38054 GRENOBLE CEDEX 09 (FR)**
• **GLIERE, Alain
38054 GRENOBLE CEDEX 09 (FR)**
• **FOURNIER, Maryse
38054 Grenoble, cedex 9 (FR)**

(74) Mandataire: **INNOV-GROUP
310, avenue Berthelot
69372 Lyon Cedex 08 (FR)**

(56) Documents cités:
WO-A1-2019/081701      US-A1- 2005 090 725
US-A1- 2014 073 899      US-A1- 2015 051 473

**EP 4 019 943 B1**

**Description**

**DOMAINE TECHNIQUE**

Le domaine technique de l'invention est la détection d'un analyte par détection photoacoustique.

**ART ANTERIEUR**

La détection photoacoustique est basée sur la détection d'une onde acoustique générée sous l'effet de l'absorption, par un milieu analysé, d'une onde incidente électromagnétique impulsionnelle ou modulée en amplitude. L'onde acoustique est formée suite à un échauffement de molécules d'intérêt, présentes dans le milieu analysé, sous l'effet de l'absorption de l'onde incidente. L'échauffement entraîne une dilatation thermique modulée du milieu, cette dernière étant à l'origine de l'onde acoustique.

La détection photoacoustique peut être spécifique à un analyte particulier, en ajustant la longueur d'onde de l'onde incidente électromagnétique à une longueur d'onde d'absorption de l'analyte. La détection photoacoustique a été ainsi été appliquée pour détecter des espèces gazeuses dans un gaz, ou pour détecter la présence de molécules particulières dans des tissus biologiques. La longueur d'onde de l'onde incidente se situe fréquemment dans l'infra-rouge.

La détection photoacoustique constitue alors une technique d'analyse non invasive, pouvant être mise en oeuvre dans des milieux diffusants ou opaques.

Des applications de détection photoacoustique à des tissus biologiques sont décrites dans les publications suivantes :

- Bauer AJ. "IR-spectroscopy for skin in vivo : optimal skin sites and properties for non-invasive glucose measurement by photoacoustic and photothermal spectroscopy" ; Journal of biophotonics 11 (2018) ;
- "Windowless ultrasound photoacoustic cell for in-vivo mid-IR spectroscopy of human epidermis : Low interférence by changes of air pressure, température, and humidity caused by skin contact opens the possibility for a non-invasive monitoring of glucose in the interstitial fluid", Rev. Sci. Instrum. 84, 084901 (2013).

Dans ces publications, on utilise une source de lumière laser modulée en amplitude, activée selon une fréquence comprise entre plusieurs dizaines de Hz et de plusieurs dizaines de kHz.

L'objectif est d'estimer une concentration de glucose dans le fluide interstitiel corporel, à une profondeur comprise entre 10 $\mu$m et 100 $\mu$m sous la surface de la peau d'un utilisateur. On utilise pour cela un dispositif de détection photoacoustique placé contre la peau d'un utilisateur.

Un dispositif de détection photoacoustique comporte un transducteur, configuré pour détecter une onde acoustique modulée en amplitude sous l'effet de l'échauffement périodique induit par l'onde lumineuse modulée. Plus précisément, le dispositif de détection photoacoustique est agencé pour détecter une modulation périodique de pression, selon une période dépendant de la fréquence de modulation de l'onde lumineuse. Une fonction de réponse du dispositif photoacoustique peut être calibrée, de façon à établir une corrélation entre la modulation de pression mesurée et la quantité d'analyte présente dans le milieu analysé.

Le document US2014/073899 décrit un dispositif de détection photoacoustique comportant une cavité destinée à être appliquée contre le corps d'un utilisateur. La cavité est remplie d'un agent de couplage imprégnant une éponge, ce qui permet une adaptation d'indice optique entre une source de lumière et le corps de l'utilisateur. Cela permet également une adaptation d'impédance acoustique entre le corps de l'utilisateur et un transducteur acoustique. Le recours à un gel de couplage, s'étendant entre la peau d'un utilisateur et un transducteur acoustique, est également décrit dans US2005/090725.

Une difficulté peut survenir du fait de la vapeur d'eau émanant de la peau, par effet de sueur. La vapeur d'eau peut se condenser et former des gouttelettes, pouvant endommager le transducteur. Par ailleurs, au cours de l'utilisation du dispositif, des poussières, ou autres éléments indésirables, par exemple des débris de peau, peuvent s'accumuler dans le dispositif. L'invention a pour objectif de résoudre ce problème.

**EXPOSE DE L'INVENTION**

Un premier objet de l'invention est un dispositif de détection photoacoustique, destiné à être appliqué, par une face de contact, contre un milieu à analyser, le dispositif comportant :

- une cavité creuse, débouchant sur une ouverture de contact, l'ouverture de contact étant ménagée dans la face de contact;
- une source de lumière, impulsionnelle ou modulée en amplitude, configurée pour émettre, lorsqu'elle est activée, un faisceau lumineux incident, dans une bande spectrale d'émission, à travers la cavité, jusqu'à l'ouverture de contact ;
- un transducteur acoustique relié à la cavité, et configuré pour détecter une onde acoustique s'étendant à travers la cavité ;

de telle sorte que sous l'effet d'une illumination du milieu, par le faisceau lumineux incident, le transducteur acoustique détecte une onde acoustique, produite par un échauffement du milieu;
le dispositif étant caractérisé en ce que :

- la cavité comporte une membrane s'étendant à travers la cavité, en regard de la face de contact ;
- la membrane est délimitée par une face inférieure et une face supérieure, la membrane comportant des ouvertures traversantes ménagées entre la face inférieure et la face supérieure;
- la membrane s'étend à l'intérieur de la cavité, à une distance non nulle de la face de contact.

Par ouverture traversante, il est entendu une ouverture permettant un passage de l'air à travers l'ouverture, entre la face inférieure et la face supérieure de la membrane.

Le dispositif peut comporter une des caractéristiques décrites ci-dessous, prises isolément ou selon les combinaisons techniquement réalisables.

- Le rayon de chaque ouverture traversante est compris entre 5 μm et 25 μm.
- La membrane définit un facteur d'ouverture, correspondant à un ratio d'une surface cumulée de chaque ouverture traversante sur la surface totale de la face inférieure ou de la face supérieure de la membrane, le facteur d'ouverture étant par exemple compris entre 0,05 et 0,3.
- L'épaisseur de la membrane est comprise entre 100 μm et 1 mm.

Avantageusement :

- la membrane est agencée de telle sorte que, lorsque la source de lumière est activée, le faisceau lumineux incident traverse la membrane avant d'atteindre l'ouverture de contact ;
- la membrane comporte une portion d'intersection, correspondant à une partie de la membrane traversée par le faisceau lumineux ;
- au moins dans la portion d'intersection, la membrane est constituée d'un matériau transparent, présentant une transmittance, dans la bande spectrale d'émission, supérieure à 0,4 et de préférence supérieure à 0,8.

La membrane peut être pleine dans la portion d'intersection. Par pleine, il est entendu sans ouverture traversante.

Le matériau transparent peut être constitué d'au moins un matériau choisi parmi : Si, Ge, AlN, ZnSe, $BaF_2$, $CaF_2$, KBr, ZnS, Saphir.

Au moins dans la portion d'intersection, la face supérieure de la membrane peut comporter un revêtement anti-reflet.

Le revêtement antireflet peut être appliqué sur toute la face supérieure, et éventuellement sur tout ou partie de la face inférieure.

Selon une possibilité, la membrane est monolithique. Elle est fabriquée à partir d'un même matériau (hors éventuel revêtement hydrophobe ou revêtement anti-reflet).

Selon une possibilité, la membrane est constituée :

- d'un premier matériau en dehors de la portion d'intersection ;
- d'un matériau auxiliaire, formant ledit matériau transparent, dans la portion d'intersection.

La membrane peut comporter un revêtement hydrophobe, notamment sur la face inférieure. Selon un mode de réalisation,

- la cavité est délimitée par une paroi transversale et une paroi latérale, la paroi latérale s'étendant entre la paroi transversale et la face de contact ;
- la membrane s'étend entre deux faces opposées de la paroi latérale.

La paroi transversale peut être parallèle à la face de contact.

Selon un mode de réalisation, la membrane est disposée de façon amovible dans la cavité.

La source de lumière peut être une source laser.

Le volume de la cavité peut être inférieur à 50 μL.

L'invention sera mieux comprise à la lecture de l'exposé des exemples de réalisation présentés, dans la suite de la description, en lien avec les figures listées ci-dessous.

## FIGURES

La figure 1A montre un mode de réalisation de dispositif de détection photoacoustique.

La figure 1B schématise une séparation de la cavité du dispositif en une cavité inférieure et une cavité supérieure.

La figure 1C schématise une membrane.

La figure 2 montre une fonction de transmission, prenant en compte la réflexion aux interfaces, d'une membrane de Silicium d'épaisseur 300 μm.

La figure 3 schématise une goutte formée sur la surface inférieure de la membrane et pénétrant dans une ouverture traversante pratiquée dans la membrane.

La figure 4A représente une cavité modélisée, dans laquelle une membrane sépare une cavité inférieure et une cavité supérieure.

La figure 4B schématise un circuit électrique équivalent de la cavité modélisée sur la figure 4A.

La figure 4C montre une amplitude de modulation de la pression dans la cavité inférieure et dans la cavité supérieure en prenant en compte deux facteurs d'ouverture de la membrane.

La figure 5A représente une variante dans laquelle la membrane comporte une partie pleine, dans une portion d'intersection correspondant à une portion de la membrane traversée par un faisceau lumineux incident.

La figure 5B schématise une variante dans laquelle la membrane est une membrane composite, formée par un matériau « standard » pas nécessairement transparent dans l'infra-rouge, et un matériau auxiliaire, transparent dans l'infra-rouge, ce dernier étant disposé dans la portion d'intersection.

## EXPOSE DE MODES DE REALISATION PARTICULIERS

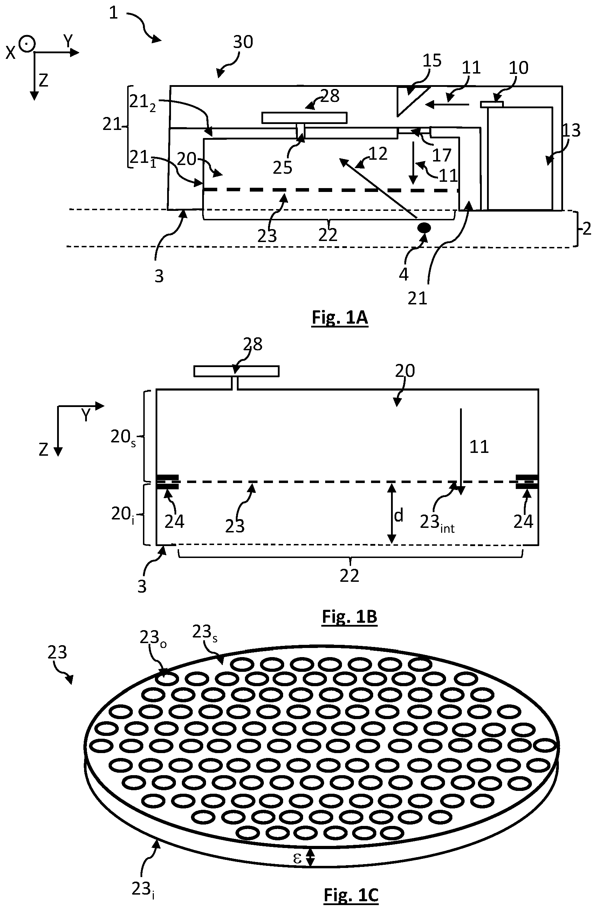

On a schématisé, sur la figure 1A, un dispositif 1 permettant une mise en oeuvre de l'invention. Le dispositif 1 est configuré pour être appliqué contre un milieu 2 à analyser. Le dispositif comporte une face de contact 3, destinée à être appliquée contre le milieu à analyser. La face de contact est conçue pour se conformer au milieu contre laquelle elle est destinée à s'appuyer. Elle est par exemple plane.

Dans cet exemple, le milieu 2 est la peau d'un utilisateur. Le dispositif comporte une source de lumière 10, configurée pour émettre un faisceau lumineux 11 se propageant jusqu'au milieu 2 à analyser. La source de lumière 10 est impulsionnelle ou modulée en amplitude. Le faisceau lumineux 11 est émis dans une bande spectrale d'émission $\Delta\lambda$ comportant une longueur d'onde d'absorption $\lambda_a$ d'un analyte 4 présent dans le milieu. Un objectif du dispositif 1 est de détecter la présence de l'analyte 4 et éventuellement d'en estimer une concentration.

L'analyte 4 peut être une molécule présente dans un fluide corporel. Il peut par exemple s'agir de glucose, ou d'un analyte corporel de type cholesterol, triglycérides, urée, albumine, alcool (par exemple éthanol), tétrahydrocannabinol.

La bande spectrale d'émission s'étend de préférence dans le visible ou dans l'infra-rouge, par exemple entre des longueurs d'onde de 3 μm et de 15 μm. De préférence, la bande spectrale d'émission $\Delta\lambda$ est suffisamment étroite, de façon que le dispositif 1 soit spécifique à un seul analyte. Lorsque l'analyte est le glucose, la bande spectrale d'émission est centrée sur une longueur d'onde d'absorption du glucose, par exemple correspondant à un nombre d'onde de 1034 cm⁻¹. La source de lumière 10 peut notamment être une source laser impulsionnelle, par exemple un laser accordable en longueur d'onde de type QCL (Quantum Cascade Laser). La bande spectrale d'émission $\Delta\lambda$ est alors située dans l'infra-rouge.

Selon d'autres modes de réalisation, la source de lumière peut être une source de type filament, ou une diode électroluminescente. Selon ces modes de réalisation, il est préférable d'associer la source de lumière à un filtre passe-bande pour définir une bande spectrale d'émission suffisamment étroite, et centrée sur la longueur d'onde d'absorption considérée. Cependant, le recours à une source laser est préféré.

Dans le mode de réalisation représentés sur la figure 1A, le dispositif 1 comporte un composant optique 15, configuré pour dévier le faisceau lumineux 11 émis par la source de lumière, vers le milieu 2 à analyser.

Le dispositif 1 est destiné à être appliqué contre le milieu à analyser 2. Il comporte une enveloppe de confinement 21, disposée au contact du milieu, et délimitant une cavité 20. La cavité 20 débouche sur ouverture de contact 22, ménagée dans la face de contact 3, de façon à déboucher sur le milieu 2. Le faisceau lumineux 11, après avoir été réfléchi par le composant optique 15, se propage jusqu'au milieu 2 à travers la cavité 20, ainsi qu'à travers l'ouverture de contact 22. Le dispositif comporte une fenêtre transparente 17, configurée pour transmettre le faisceau lumineux incident 11.

Dans le dispositif représenté sur la figure 1A, le composant optique 15 est un réflecteur, prenant la forme d'un prisme réfléchissant. De préférence, le faisceau lumineux incident 11 parvient jusqu'au milieu 2 selon une incidence normale, ou sensiblement normale. Par sensiblement normal, on entend normal en considérant une tolérance angulaire de ± 30 °.

Sous l'effet de la présence d'un analyte 4 dans le milieu 2, une onde acoustique, dite onde photoacoustique 12, est formée. L'onde photoacoustique 12 est une onde acoustique formée à partir d'un échauffement périodique du milieu par le faisceau lumineux incident 11, ce dernier étant impulsionnel ou modulé en amplitude. Une partie de l'onde photoacoustique 12 s'étend à travers la cavité 20 de façon à être détectée par un transducteur acoustique 28. Le transducteur acoustique 28 est relié à la cavité 20 par un canal acoustique 25. Le transducteur acoustique peut être un microphone, présentant une gamme spectrale de détection comprenant la fréquence de l'onde photoacoustique. L'onde photoacoustique est modulée en amplitude selon la fréquence d'impulsion ou de modulation d'amplitude de la source de lumière. Ainsi, au niveau du transducteur, la pression est modulée en amplitude.

L'enveloppe de confinement 21 comporte :

- une composante latérale $21_1$, s'étendant de préférence parallèlement à un axe normal Z à la face de contact 3. La composante latérale $21_1$ forme une paroi latérale délimitant la cavité.
- une composante transversale $21_2$, s'étendant parallèlement, ou sensiblement parallèlement à la face de contact 3, en regard de cette dernière. La composante transversale $21_2$ s'étend parallèlement, ou sensiblement parallèlement à l'ouverture de contact 22. Dans le mode de réalisation représenté sur la figure 1A, la composante transversale $21_2$ inclut la fenêtre 17. La composante transversale $21_2$ forme une paroi transversale délimitant la cavité.

Par sensiblement parallèlement, on entend parallèle en admettant une tolérance angulaire de ± 30° ou ± 20°.

La face latérale $21_1$ s'étend entre la face de contact 3 et la face transversale $21_2$.

Le dispositif comporte un capot de protection 30, enveloppant les composants précédemment décrits. La source de lumière est disposée sur un support 13, relié au capot 30.

Comme mentionné dans la publication Kottmann "Mid-infrared photoacoustic détection of glucose in human skin : towards non-invasive diagnostics", Sensors 2016, 16, 1663, une relation peut être établie, entre l'amplitude $A$ de modulation de l'onde photoacoustique, à la fréquence de modulation $f$ et le volume $V$ de la cavité 20, de telle sorte que :

$$ A \propto \frac{I_{11}(\lambda)\alpha(\lambda)}{V f^{\frac{3}{2}}} \ (1) $$

où :

- $\propto$ est l'opérateur proportionnel ;
- $I_{11}(\lambda)$ est l'intensité du faisceau lumineux incident à la longueur d'onde $\lambda$ ;
- $\alpha(\lambda)$ est un coefficient d'absorption du milieu analysé à la longueur d'onde $\lambda$ ;
- $V$ est le volume de la cavité, incluant éventuellement le canal acoustique;
- $f$ est la fréquence de modulation de l'onde acoustique.

Lorsque la fréquence $f$ et l'intensité du faisceau lumineux $I_{11}(\lambda)$ sont fixées, l'amplitude de modulation de l'onde photoacoustique $A$, détectée par le transducteur acoustique est proportionnelle au coefficient d'absorption du milieu $\alpha(\lambda)$. Or, ce dernier est considéré comme proportionnel à la concentration d'analyte dans le milieu. Ainsi, la mesure de l'amplitude de modulation $A$ par le transducteur acoustique 28 permet une estimation de la concentration d'analyte 4 dans le milieu, via une prise en compte du coefficient d'absorption du milieu $\alpha(\lambda)$.

Le dispositif comporte une membrane 23, s'étendant à l'intérieur de la cavité 20, entre la face de contact 3 et le transducteur 28. Comme illustré sur la figure 1B, la membrane 23 permet une segmentation de la cavité entre :

- une cavité inférieure $20_i$, s'étendant entre la face de contact 3 et la membrane 23 ;
- une cavité supérieure $20_s$, s'étendant entre la membrane 23 et le transducteur 28.

Ainsi, la membrane forme un écran de protection, interposé entre la cavité inférieure $20_i$ et la cavité supérieure $20_s$. Cela permet d'isoler la cavité supérieure $20_s$ de gouttelettes d'eau ou de poussières, ou autres éléments indésirables susceptibles d'être présents dans la cavité inférieure $20_i$, en ayant accédé à cette dernière à travers l'ouverture de contact 22.

La membrane 23 s'étend à l'intérieur de la cavité 20, à une distance d non nulle de l'ouverture de contact 22. En effet, lors de la mise en oeuvre du dispositif, il est préférable que le membrane 23 ne soit pas en contact avec la peau 2, afin de ne pas perturber l'échauffement d'une couche superficielle de gaz au contact de la peau 2. Le fait de disposer la membrane à distance permet de maintenir une couche d'air entre l'ouverture de contact 22 et la membrane. La distance entre la membrane et l'ouverture de contact est de préférence supérieure à 200 $\mu$m, ou 500 $\mu$m.

La membrane s'étend de préférence à travers toute la cavité, en regard de la face de contact 3. Elle s'étend entre des points opposés de la face latérale. La membrane est de préférence disposée parallèlement à la face de contact, ou sensiblement parallèlement à cette dernière.

La membrane 23 est maintenue à l'intérieur de la cavité 20, par un support 24. Dans cet exemple, la membrane est insérée dans le support 24. La membrane 23 peut être amovible, ce qui permet un remplacement et/ou un nettoyage de cette dernière.

Lorsque la source de lumière 10 est activée, le faisceau lumineux 11 traverse la membrane 23 avant d'atteindre l'ouverture de contact 22. La membrane comporte une portion d'intersection $23_{int}$, correspondant à la partie de la membrane traversée par le faisceau lumineux 11.

Au moins dans la portion d'intersection $23_{int}$, la membrane est formée d'un matériau présentant une transmittance élevée dans la bande spectrale $\Delta\lambda$ du faisceau d'émission 11. Par transmittance élevée, on entend une transmittance du matériau de préférence supérieure à 0,4 voire et de préférence supérieure à 0,8, par exemple de l'ordre de 0,9 ou davantage. Le matériau peut par exemple être le silicium. Par transmittance, on entend une fraction de l'intensité lumineuse transmise par la membrane 23. La membrane peut être partiellement ou entièrement formée de Si, ou autre matériau transparent à l'infra-rouge, par exemple Si poreux, Ge, AlN, ZnSe, $BaF_2$, $CaF_2$, KBr, ZnS, Saphir.

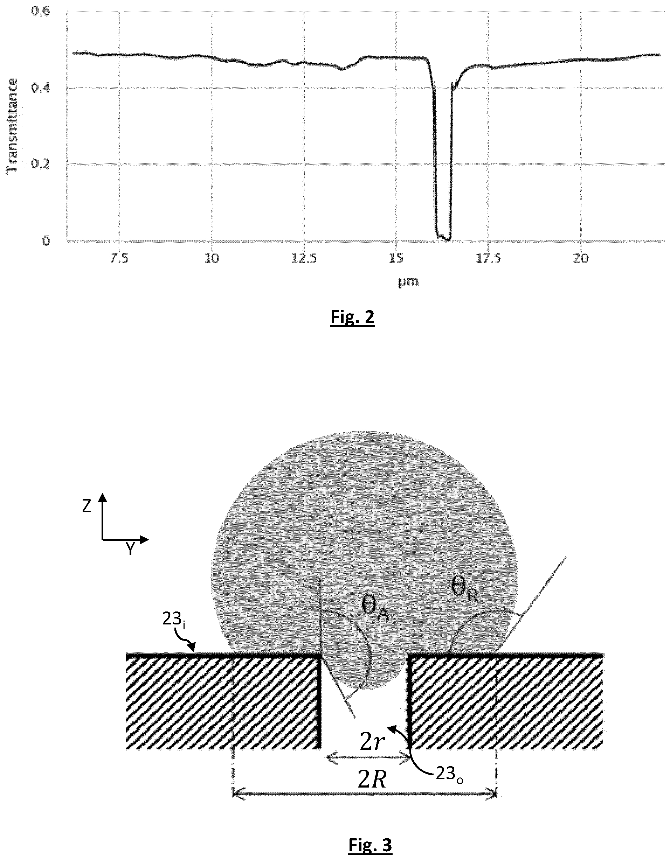

La figure 2 représente la transmittance (axe des ordonnées), en fonction de la longueur d'onde (axes des abscisses - unité $\mu$m), d'une membrane de Si d'épaisseur 300 $\mu$m. La transmittance est affectée par les réflexions, notamment au niveau de la face supérieure $23_s$. La transmittance peut être augmentée, pour atteindre des valeurs voisines de 1, en appliquant un revêtement anti-reflet, en particulier sur la face supérieure $23_s$ et de préférence la face supérieure $23_s$ et la face inférieure $23_i$. Le revêtement antireflet peut prendre la forme d'une couche pleine « quart d'onde », déposée sous la forme de couche mince. Le dépôt de la couche mince peut avoir lieu sur tout ou partie de la face supérieure (et de préférence de la face inférieure), sans risque de boucher les ouvertures traversantes, du fait de la faible épaisseur de la couche mince.

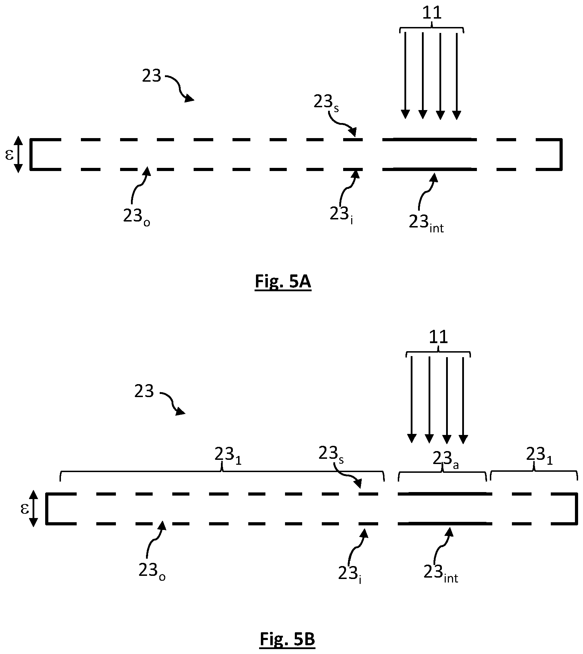

La membrane peut également être composite, en comportant un matériau considéré comme suffisamment transparent à l'infra-rouge au niveau de la portion d'intersection $23_{int}$, d'un autre matériau en dehors de la portion d'intersection. Un exemple de membrane composite est décrit par la suite en lien avec la figure 5B.

Afin de permettre une transmission des modulations de pression à travers la cavité 20, jusqu'au transducteur 28, la membrane comporte des ouvertures traversantes $23_o$, s'étendant à travers toute l'épaisseur de la membrane. Les ouvertures traversantes sont représentées sur la figure 1C. Les ouvertures traversantes sont dimensionnées pour transmettre la modulation de pression à travers la membrane 23, tout en bloquant des gouttes de liquide ou des poussières. Ces ouvertures traversantes 23 permettent une communication d'air entre la cavité inférieure $20_i$ et la cavité supérieure $20_s$.

La figure 3 représente une goutte déposée sur la face inférieure $23_i$ de la membrane 23. On a représenté des angles de mouillage $\theta_R$ et $\theta_A$ de la goutte respectivement sur la face inférieure de la membrane et dans une ouverture traversante $23_o$. On considère ici une microgoutte, pouvant se former par condensation de vapeur d'eau résultant de la sueur. La goutte étant supposée microscopique, les forces de capillarité prédominent par rapport aux forces de gravité. La goutte subit une différence de pression de part et d'autre de la membrane, due à des forces de capillarité antagonistes, qui interagissent sur la goutte au niveau de la face inférieure $23_i$, et au niveau de l'ouverture traversante $23_o$. Les forces de capillarité induisent une différence de pression $\Delta p$ subie par la goutte pouvant être explicitée par :

$$\Delta p = -2\gamma \frac{\cos(\theta_A)}{r} + 2\gamma \frac{\cos(\theta_R)}{R} \quad (2)$$

où

- $\gamma$ : tension superficielle liquide / air ; lorsque le liquide est de l'eau, $\gamma = 0,073$ N/m ; lorsque le liquide est un tampon biologique, davantage représentatif de la sueur, $\gamma = 0,03$ N/m
- $r$ : rayon de l'ouverture traversante $23_o$ ;
- $R$ : rayon de la ligne mouillée sur la face inférieure $23_i$.

L'expression (2) est issue de Cho, H.-Y. Kim, J. Y. Kang, et T. S. Kim, « How the capillary burst microvalve works », J. Colloid Interface Sci., vol. 306, n° 2, p. 379-385, février 2007. L'expression (2) définit une condition de pénétration de la goutte dans une ouverture traversante de section circulaire. La membrane bloque la goutte lorsque $\Delta p > 0$.

La goutte forme un ménisque, qui s'engage dans l'ouverture traversante $23_o$, et est soumis à des forces de capillarité tendant à faire progresser la goutte à l'intérieur d'un capillaire formé par l'ouverture traversante. La pression résultante est $-2\gamma \frac{\cos(\theta_A)}{r}$. Une partie résiduelle de la goutte est retenue sur la face inférieure $23_i$, et est soumis à des forces de capillarité. La pression résultante est $2\gamma \frac{\cos(\theta_R)}{R}$.

Afin d'augmenter l'angle de mouillage $\theta_R$, il est possible d'appliquer un traitement de surface hydrophobe sur la face inférieure $23_i$ de la membrane. En effet, lorsque le matériau formant la membrane est Si, qui est un matériau hydrophile, l'angle de mouillage pour l'eau est de 5°. Lorsque l'on considère une goutte d'un tampon biologique, ce qui s'approche davantage des conditions rencontrées en appliquant le dispositif sur la peau d'un utilisateur, l'angle de mouillage est de l'ordre de 20° à 40°. L'application d'un traitement de surface hydrophobe, par exemple de type silanisation (greffage de fonctions silanes hydrophobes), permet d'augmenter l'angle de mouillage à 110° pour l'eau et 80° pour le tampon biologique. Un traitement de surface hydrophobe renforce ainsi la capacité de rétention de la goutte sur la face inférieure de la membrane. Le traitement hydrophobe peut également « déborder » jusqu'à la surface interne des ouvertures traversantes.

Outre la mouillabilité du liquide, la tension de surface $\gamma$ est également un paramètre déterminant. Lorsque le diamètre d'une ouverture traversante $23_o$ est égal à 20 μm ($r = 10$ μm), et que le liquide est respectivement de l'eau ($\gamma = 0,073$ N/m), ou du liquide biologique ($\gamma = 0,03$ N/m), l'application de l'expression (1) conduit à $\Delta p = 0.14$ bar et $\Delta p = 0.06$ bar. Il faut donc appliquer une surpression supérieure à $\Delta p$ pour que la goutte franchisse la membrane par capillarité. Cette estimation a été effectuée en considérant R = 20 μm .

Le rayon des ouvertures traversantes est de préférence compris entre 5 μm et 25 μm, et de préférence entre 5 μm et 15 μm. Lorsque le rayon augmente, la transmission des modulations de pression est optimale, mais la valeur $\Delta p$ diminue : la membrane est moins apte à bloquer un passage de gouttelettes à travers les ouvertures traversantes. Cet inconvénient peut, dans une certaine mesure, être surmonté en appliquant un traitement de surface hydrophobe sur la face inférieure $23_i$.

L'épaisseur $\epsilon$ de la membrane 23 est de préférence comprise entre 100 μm et 1 mm, et de préférence entre 150 μm et 750 μm.

Le rayon de chaque ouverture traversante dépend également de l'épaisseur $\epsilon$ de la membrane. Les ouvertures traversantes peuvent être formées, à partir d'un substrat de Si, par une photolithographie suivie d'une gravure humide. Dans ce cas, il est considéré qu'on peut former des ouvertures traversantes dont le diamètre est de l'ordre du dixième de l'épaisseur $\epsilon$, voire moins si

nécessaire.

La membrane est dimensionnée pour permettre une transmission de la modulation de pression entre les parties inférieure et supérieure de la cavité. Le nombre d'ouvertures traversantes doit être déterminé de façon que l'effet de la membrane sur l'onde photoacoustique puisse être considéré comme négligeable, dans la plage fréquentielle correspondant à la fréquence d'impulsion de la source de lumière.

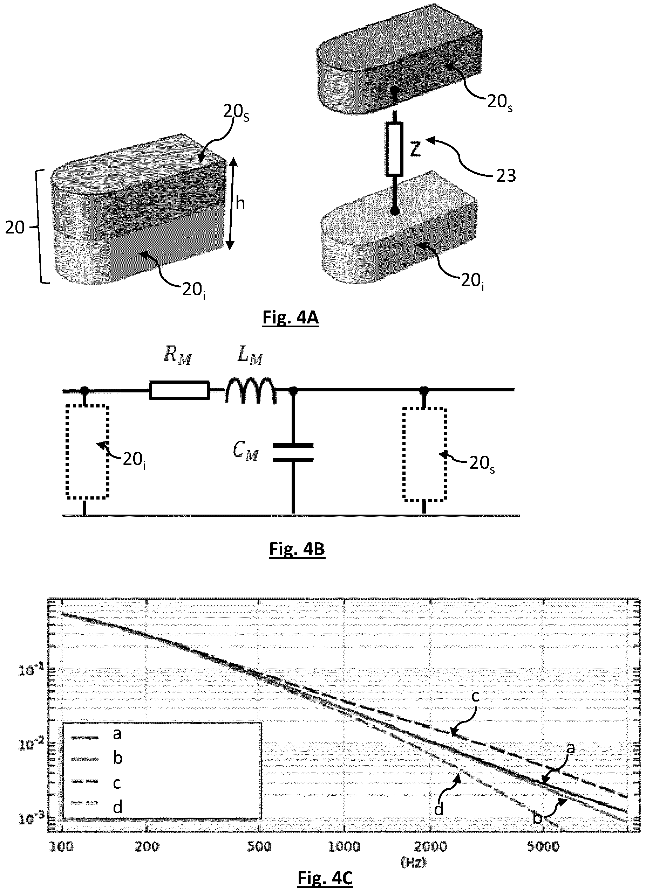

Le facteur d'ouverture de la membrane correspond à un ratio entre la surface cumulée de chaque ouverture traversante sur la surface totale de la face inférieure (ou de la face supérieure). Le facteur d'ouverture peut être compris entre 0,01 et 0,3. Les inventeurs ont modélisé la transmission des modulations d'amplitude de l'onde photoacoustique 12 pour deux facteurs d'ouverture. Le modèle a été élaboré en considérant que la membrane constitue une impédance acoustique analogue à une impédance électrique. La figure 4A représente la cavité 20 modélisée (figure de gauche), la membrane 23, disposée à mi-hauteur, formant une impédance acoustique assimilable à une impédance électrique Z (figure de droite). L'impédance acoustique a été modélisée par un circuit RLC tel que représenté sur la figure 4B. La membrane est assimilée à un circuit $R_M$, $L_M$, $C_M$, entre la cavité inférieure $20_i$ et la cavité supérieure $20_s$.

La cavité modélisée présentait un volume de 4,45 mm$^3$ et une hauteur h de 1,5 mm. On a considéré deux facteurs d'ouverture différents :

- un premier facteur d'ouverture, correspondant à une disposition de 1000 ouvertures traversantes de rayon 10 μm : la valeur du premier facteur d'ouverture est de 0,1 ;
- un deuxième facteur d'ouverture, correspondant à une disposition de 100 ouvertures traversantes de rayon 10 μm : la valeur du deuxième facteur d'ouverture est de 0,01.

L'épaisseur de la membrane modélisée était de 200 μm.

La figure 4C représente l'amplitude d'une modulation de la pression (axe des ordonnées - unités arbitraires) en fonction de la fréquence de modulation (axe des abscisses - Hz) :

- en considérant le premier facteur d'ouverture, dans la cavité inférieure $20_i$ (courbe a - trait plein noir) et dans la cavité supérieure $20_s$ (courbe b - trait plein gris) ;
- en considérant le deuxième facteur d'ouverture, dans la cavité inférieure $20_i$ (courbe c - tirets noirs) et dans la cavité supérieure $20_s$ (courbe d - tirets gris).

La figure 4C montre l'effet du facteur d'ouverture sur la transmission de la modulation de pression de part et d'autre de la membrane. On observe que le deuxième facteur d'ouverture, plus faible, entraîne une atténuation de la modulation de pression transmise par la membrane, en particulier aux fréquences élevées.

Afin d'éviter que la transmission du faisceau lumineux 11 subisse des effets de diffraction, la portion d'intersection $23_{int}$ de la membrane peut être pleine, comme représenté sur la figure 5A. La portion pleine peut comporter un revêtement antireflet, appliqué sur la face supérieure $23_s$ et de préférence également sur la face inférieure $23_i$. Le revêtement antireflet peut être une couche mince ou un cristal photonique. Par portion pleine, il est entendu une portion ne comportant pas d'ouverture traversante.

Le membrane peut être monolithique, c'est-à-dire formée d'un même matériau, hors éventuel traitement antireflet ou éventuel traitement hydrophobe. La figure 5B montre une variante, selon laquelle la membrane est une membrane composite. La membrane est composée d'un premier matériau $23_1$, standard, pas nécessairement transparent dans l'infra-rouge, en dehors de la portion d'intersection. Au niveau de la portion d'intersection, la membrane comporte un matériau auxiliaire $23_a$, transparent dans l'infra-rouge. Le premier matériau $23_1$ peut être celui d'une membrane poreuse standard, par exemple un matériau de type GoreTex (marque déposée). Le matériau auxiliaire $23_a$ est différent du premier matériau $23_1$.

**Revendications**

1. Dispositif de détection photoacoustique (1), destiné à être appliqué, par une face de contact (3), contre un milieu à analyser (2), le dispositif comportant :

   - une cavité creuse (20), débouchant sur une ouverture de contact (22), l'ouverture de contact étant ménagée dans la face de contact;
   - une source de lumière (10), impulsionnelle ou modulée en amplitude, configurée pour émettre, lorsqu'elle est activée, un faisceau lumineux incident (11), dans une bande spectrale d'émission (Δλ), à travers la cavité (20), jusqu'à l'ouverture de contact ;
   - un transducteur acoustique (28) relié à la cavité, et configuré pour détecter une onde acoustique (12) s'étendant à travers la cavité ;

   de telle sorte que, sous l'effet d'une illumination du milieu par le faisceau lumineux incidente, le transducteur acoustique détecte une onde acoustique produite par un échauffement du milieu (2); le dispositif étant **caractérisé en ce que** :

   - la cavité comporte une membrane s'étendant à travers la cavité, en regard de la face de contact ;
   - la membrane est délimitée par une face infé-

rieure ($23_i$) et une face supérieure ($23_s$), la membrane comportant des ouvertures traversantes ($23_o$) ménagées entre la face inférieure et la face supérieure ;
- la membrane s'étend à l'intérieur de la cavité, à une distance (d) non nulle de la face de contact (3).

2. Dispositif selon la revendication 1, dans lequel le rayon de chaque ouverture traversante ($23_o$) est compris entre 5 $\mu$m et 25 $\mu$m.

3. Dispositif selon l'une quelconque des revendications précédentes, dans lequel :

- la membrane définit un facteur d'ouverture, correspondant à un ratio d'une surface cumulée de chaque ouverture traversante sur la surface totale de la face inférieure ou de la face supérieure de la membrane ;
- le facteur d'ouverture est compris entre 0,05 et 0,3.

4. Dispositif selon l'une quelconque des revendications précédentes, dans lequel l'épaisseur de la membrane est comprise entre 100 $\mu$m et 1 mm.

5. Dispositif selon l'une quelconque des revendications précédentes, dans lequel :

- la membrane est agencée de telle sorte que, lorsque la source de lumière est activée, le faisceau lumineux incident traverse la membrane avant d'atteindre l'ouverture de contact (22);
- la membrane comporte une portion d'intersection ($23_{int}$), correspondant à une partie de la membrane traversée par le faisceau lumineux ;
- au moins dans la portion d'intersection, la membrane est constituée d'un matériau transparent, présentant une transmittance, dans la bande spectrale d'émission, supérieure à 0,4.

6. Dispositif selon la revendication 5, dans lequel la membrane est pleine dans la portion d'intersection.

7. Dispositif selon l'une quelconque des revendications 5 ou 6, dans lequel le matériau transparent est constitué d'au moins un matériau choisi parmi : Si, Ge, AlN, ZnSe, $BaF_2$, $CaF_2$, KBr, ZnS, Saphir.

8. Dispositif selon l'une quelconque des revendications 5 à 7 dans lequel au moins dans la portion d'intersection ($23_{int}$), la face supérieure ($23_s$) de la membrane comporte un revêtement anti-reflet.

9. Dispositif selon l'une quelconque des revendications 5 à 8 dans lequel la membrane est constituée :

- d'un premier matériau ($23_1$) en dehors de la portion d'intersection ;
- d'un matériau auxiliaire ($23_a$), formant ledit matériau transparent, dans la portion d'intersection.

10. Dispositif selon l'une quelconque des revendications précédentes, dans lequel la membrane comporte un revêtement hydrophobe sur la face inférieure.

11. Dispositif selon l'une quelconque des revendications précédentes, dans lequel :

- la cavité est délimitée par une paroi transversale ($21_2$) et une paroi latérale ($21_1$), la paroi latérale s'étendant entre la paroi transversale et la face de contact ;
- la membrane s'étend entre deux faces opposées de la paroi latérale.

12. Dispositif selon l'une quelconque des revendications précédentes, dans lequel la membrane est disposée de façon amovible dans la cavité.

13. Dispositif selon l'une quelconque des revendications précédentes, dans lequel la source de lumière est une source laser.

14. Dispositif selon l'une quelconque des revendications précédentes, dans lequel le volume de la cavité est inférieur à 50 $\mu$L.


**Patentansprüche**

1. Photoakustische Detektionsvorrichtung (1), die dazu bestimmt ist, mit einer Kontaktseite (3) an ein zu analysierendes Milieu (2) angelegt zu werden, wobei die Vorrichtung umfasst:

- einen Hohlraum (20), der auf einer Kontaktöffnung (22) mündet, wobei die Kontaktöffnung in der Kontaktseite ausgebildet ist;
- eine impulsartige oder amplitudenmodulierte Lichtquelle (10), die dazu ausgestaltet ist, wenn sie aktiviert ist, einen einfallenden Lichtstrahl (11), in einem Emissionsspektralband ($\Delta\lambda$), durch den Hohlraum (20) hindurch bis zu der Kontaktöffnung auszusenden;
- einen Schallwandler (28), der mit dem Hohlraum verbunden ist und dazu ausgestaltet ist, eine Schallwelle (12) zu detektieren, die sich durch den Hohlraum hindurch erstreckt;

so dass, unter der Wirkung einer Beleuchtung des Milieus durch den einfallenden Lichtstrahl, der Schallwandler eine Schallwelle detektiert, die durch eine Erwärmung des Milieus (2) erzeugt wird;

wobei die Vorrichtung **dadurch gekennzeichnet ist, dass**:

- der Hohlraum eine Membran umfasst, die sich durch den Hohlraum hindurch gegenüber der Kontaktseite erstreckt;
- die Membran durch eine Unterseite ($23_i$) und eine Oberseite ($23_s$) begrenzt wird, wobei die Membran durchgehende Öffnungen ($23_o$) umfasst, die zwischen der Unterseite und der Oberseite ausgebildet sind;
- die Membran sich im Inneren des Hohlraums erstreckt, in einem Abstand (d) ungleich null von der Kontaktseite (3).

2. Vorrichtung nach Anspruch 1, wobei der Radius jeder durchgehenden Öffnung ($23_o$) zwischen 5 $\mu$m und 25 $\mu$m beträgt.

3. Vorrichtung nach einem der vorhergehenden Ansprüche, bei der:

- die Membran einen Öffnungsfaktor definiert, der einem Verhältnis einer kumulierten Fläche jeder durchgehenden Öffnung zur Gesamtfläche der Unterseite oder der Oberseite der Membran entspricht;
- der Öffnungsfaktor zwischen 0,05 und 0,3 beträgt.

4. Vorrichtung nach einem der vorhergehenden Ansprüche, bei der die Dicke der Membran zwischen 100 $\mu$m und 1 mm beträgt.

5. Vorrichtung nach einem der vorhergehenden Ansprüche, bei der:

- die Membran so gestaltet ist, dass, wenn die Lichtquelle aktiviert ist, der einfallende Lichtstrahl die Membran durchquert, bevor er die Kontaktöffnung (22) erreicht;
- die Membran einen Grenzflächenabschnitt ($23_{int}$) umfasst, der einem Teil der Membran entspricht, der von dem Lichtstrahl durchquert wird;
- mindestens in dem Grenzflächenabschnitt die Membran aus einem transparenten Material besteht, das einen Transmissionsgrad, im Emissionsspektralband, von mehr als 0,4 aufweist.

6. Vorrichtung nach Anspruch 5, wobei die Membran in dem Grenzflächenabschnitt durchgehend ist.

7. Vorrichtung nach einem der Ansprüche 5 oder 6, bei der das transparente Material aus mindestens einem Material besteht, das gewählt ist unter: Si, Ge, AIN, ZnSe, $BaF_2$, $CaF_2$, KBr, ZnS, Saphir.

8. Vorrichtung nach einem der Ansprüche 5 bis 7, bei der mindestens in dem Grenzflächenabschnitt ($23_{int}$) die Oberseite ($23_s$) der Membran eine Antireflexbeschichtung umfasst.

9. Vorrichtung nach einem der Ansprüche 5 bis 8, bei der die Membran aus Folgendem besteht:

- einem ersten Material ($23_i$) außerhalb des Grenzflächenabschnitts;
- einem Hilfsmaterial ($23_a$), welches das transparente Material bildet, in dem Grenzflächenabschnitt.

10. Vorrichtung nach einem der vorhergehenden Ansprüche, bei der die Membran eine hydrophobe Beschichtung auf der Unterseite umfasst.

11. Vorrichtung nach einem der vorhergehenden Ansprüche, bei der:

- der Hohlraum durch eine Querwand ($21_2$) und eine Seitenwand ($21_1$) begrenzt wird, wobei sich die Seitenwand zwischen der Querwand und der Kontaktseite erstreckt;
- die Membran sich zwischen zwei entgegengesetzten Seiten der Seitenwand erstreckt.

12. Vorrichtung nach einem der vorhergehenden Ansprüche, bei der die Membran in dem Hohlraum lösbar angeordnet ist.

13. Vorrichtung nach einem der vorhergehenden Ansprüche, bei der die Lichtquelle eine Laserquelle ist.

14. Vorrichtung nach einem der vorhergehenden Ansprüche, bei der das Volumen des Hohlraums weniger als 50 $\mu$L beträgt.

**Claims**

1. Photoacoustic detecting device (1) intended to be applied, via a contact face (3), against a medium (2) to be analysed, the device comprising:

- a hollow cavity (20) that opens onto a contact aperture (22), the contact aperture being produced in the contact face;
- a pulsed or amplitude-modulated light source (10) configured to emit, when it is activated, an incident light beam (11), in an emission spectral band ($\Delta\lambda$), through the cavity (20), to the contact aperture;
- an acoustic transducer (28) connected to the cavity, and configured to detect an acoustic wave (12) extending through the cavity;

such that, under the effect of an illumination of the medium by the incident light beam, the acoustic transducer detects an acoustic wave produced by heating of the medium (2);
the device being **characterized in that**:

- the cavity comprises a membrane extending through the cavity, facing the contact face;
- the membrane is bounded by a lower face ($23_i$) and an upper face ($23_s$), the membrane comprising through-apertures ($23_o$) produced between the lower face and the upper face;
- the membrane lies inside the cavity, at a nonzero distance (d) from the contact face (3).

2. Device according to Claim 1, wherein the radius of each through-aperture ($23_o$) is comprised between 5 $\mu$m and 25 $\mu$m.

3. Device according to either one of the preceding claims, wherein:

- the membrane defines an aperture factor, corresponding to a ratio of a cumulative area of each through-aperture to the total area of the lower face or of the upper face of the membrane;
- the aperture factor is comprised between 0.05 and 0.3.

4. Device according to any one of the preceding claims, wherein the thickness of the membrane is comprised between 100 $\mu$m and 1 mm.

5. Device according to any one of the preceding claims, wherein:

- the membrane is arranged such that, when the light source is activated, the incident light beam passes through the membrane before reaching the contact aperture (22);
- the membrane comprises an intersection segment ($23_{int}$), corresponding to a portion of the membrane passed through by the light beam;
- at least in the intersection segment, the membrane is made of a transparent material, having a transmittance, in the emission spectral band, higher than 0.4.

6. Device according to Claim 5, wherein the membrane is unapertured in the intersection segment.

7. Device according to either one of Claims 5 and 6, wherein the transparent material consists of at least one material chosen from: Si, Ge, AlN, ZnSe, $BaF_2$, $CaF_2$, KBr, ZnS, sapphire.

8. Device according to any one of Claims 5 to 7, wherein at least in the intersection segment ($23_{int}$), the upper face ($23_s$) of the membrane comprises an antireflection coating.

9. Device according to any one of Claims 5 to 8, wherein the membrane is made:

- of a first material ($23_1$) outside of the intersection segment;
- of an auxiliary material ($23_a$), forming said transparent material, in the intersection segment.

10. Device according to any one of the preceding claims, wherein the membrane comprises a hydrophobic coating on the lower face.

11. Device according to any one of the preceding claims, wherein:

- the cavity is bounded by a transverse wall ($21_2$) and a lateral wall ($21_1$), the lateral wall extending between the transverse wall and the contact face;
- the membrane extends between two opposite faces of the lateral wall.

12. Device according to any one of the preceding claims, wherein the membrane is placed removably in the cavity.

13. Device according to any one of the preceding claims, wherein the light source is a laser source.

14. Device according to any one of the preceding claims, wherein the volume of the cavity is smaller than 50 $\mu$L.

**Fig. 1A**

**Fig. 1B**

**Fig. 1C**

**Fig. 2**

**Fig. 3**

**Fig. 4A**

**Fig. 4B**

**Fig. 4C**

11

23

23$_s$

$\varepsilon$

23$_o$

23$_i$

23$_{int}$

**Fig. 5A**

11

23

23$_1$

23$_s$

23$_a$

23$_1$

$\varepsilon$

23$_o$

23$_i$

23$_{int}$

**Fig. 5B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2014073899 A

- US 2005090725 A

**Littérature non-brevet citée dans la description**

- **BAUER AJ.** IR-spectroscopy for skin in vivo : optimal skin sites and properties for non-invasive glucose measurement by photoacoustic and photothermal spectroscopy. *Journal of biophotonics,* 2018, vol. 11
- Windowless ultrasound photoacoustic cell for in-vivo mid-IR spectroscopy of human epidermis : Low interférence by changes of air pressure, température, and humidity caused by skin contact opens the possibility for a non-invasive monitoring of glucose in the interstitial fluid. *Rev. Sci. Instrum.,* 2013, vol. 84, 084901

- **KOTTMANN.** Mid-infrared photoacoustic détection of glucose in human skin : towards non-invasive diagnostics. *Sensors,* 2016, vol. 16, 1663
- **CHO, H.-Y. KIM ; J. Y. KANG ; T. S. KIM.** How the capillary burst microvalve works. *J. Colloid Interface Sci.,* Février 2007, vol. 306 (2), 379-385